(19) **Europäisches Patentamt / European Patent Office / Office européen des brevets**

(11) **EP 2 493 523 B1**

(12) **EUROPEAN PATENT SPECIFICATION**

(45) Date of publication and mention of the grant of the patent:
**18.06.2014 Bulletin 2014/25**

(21) Application number: **10779734.2**

(22) Date of filing: **29.10.2010**

(51) Int Cl.:
*A61L 31/02* (2006.01)     *A61L 31/14* (2006.01)
*A61L 27/04* (2006.01)     *A61L 27/58* (2006.01)

(86) International application number:
**PCT/EP2010/066431**

(87) International publication number:
**WO 2011/051424 (05.05.2011 Gazette 2011/18)**

(54) **BIODEGRADABLE IMPLANTABLE MEDICAL DEVICES FORMED FROM SUPER - PURE MAGNESIUM-BASED MATERIAL**

BIOLOGISCH ABBAUBARE IMPLANTIERBARE MEDIZINISCHE VORRICHTUNGEN AUS SUPERREINEM MATERIAL AUF MAGNESIUMBASIS

DISPOSITIFS MÉDICAUX IMPLANTABLES BIODÉGRADABLES FORMÉS À PARTIR D'UN MATÉRIAU À BASE DE MAGNÉSIUM SUPER PUR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.10.2009 EP 09174604**
**30.10.2009 US 256496 P**

(43) Date of publication of application:
**05.09.2012 Bulletin 2012/36**

(73) Proprietor: **Acrostak Corp BVI, Tortola**
**8409 Winterthur (CH)**

(72) Inventors:
• **PAPIROV, Igor Isakovich**
**Kharkov 61108 (UA)**

• **PIKALOV, Anatoliy Ivanovich**
**Kharkov 61108 (UA)**
• **SIVTSOV, Sergey Vladimirovich**
**Kharkov 61034 (UA)**
• **SHOKUROV, Vladimir Sergeevich**
**Kharkov 61004 (UA)**
• **POPOWSKI, Youri**
**CH-1206 Geneva (CH)**

(74) Representative: **Gyi, Jeffrey Ivan et al**
**De Clercq & Partners cvba**
**Edgard Gevaertdreef 10 a**
**9830 Sint-Martens-Latem (BE)**

(56) References cited:
**EP-A1- 1 835 043     EP-A1- 2 000 551**
**US-A- 5 698 158**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**FIELD OF THE INVENTION**

The present invention generally relates to an implantable medical device, in particular, a biodegradable endoprosthesis body such as a vessel stent, formed at least partly from a constructional material comprising super-pure magnesium or an alloy thereof further comprising one or more super-pure alloying elements. The super-pure magnesium-based constructional material may be incorporated into an implantable biodegradable endoprosthesis as such, and used in various technical fields.

**BACKGROUND OF THE INVENTION**

In recent years, an interest in biodegradable (biocorrodible, bioabsorbable etc.) endoprosthesises has been observed worldwide. By definition, such devices are capable of being slowly dissolved in living body liquids and completely disappearing over time providing they have the optimum corrosion resistance. Dissolution is concurrent with performance of their medical function and avoids undesirable consequences of their presence in an organism as alien body. By contrast, implanting *in vivo* a "permanent" endoprosthesis made of insoluble material will eventually require surgical re-intervention for its extraction (*e.g.* bone or coronary surgery), otherwise its continued presence will increase the probability of adverse consequences for a patient such as inflammation, aneurysm, in-stent restenosis or thrombosis etc) in case of vascular stents. Therefore, the interest in biodegradable technology applied to endoprosthesises is of relevance to patient care and effectiveness of treatment.

There are a number of early examples of biomaterials for endoprosthesises manufacture. One of such examples describes [1]: *"...A vessel wall support ..., wherein the first component is at least one metal selected from the group consisting of magnesium, titanium, zirconium, niobium, tantalum, zinc and silicon and the second component is at least one metal selected from the group consisting of lithium, sodium, potassium, manganese calcium and iron".* Later, biodegradable metal constructional materials were employed formed as from pure (not alloyed) metals, which included Iron [3-5], Zinc, Magnesium and Molybdenum [5, 6], so and alloys: iron-alloys [5-8], zinc-alloys [5, 6], tungsten-alloys [6] and others. However, researchers subsequently placed emphasis on magnesium alloys as having the most promising characteristics for biodegradable , materials. It is known that magnesium is one of the most important elements in the life cycle of a living body and influences metabolism [9]; magnesium ions are the fourth most abundant metal ions the human body. It is known that an adult man consumes daily 300-400 mg of magnesium; to put this into context, a magnesium stent has a weight only about of 1 mg, consequently, its degradation should not influence magnesium content in the living body. Recently, it has been reported that the presence of magnesium in the human bone structure is beneficial to bone strength and growth [10]. Magnesium alloys have a specific density (1.7 - 1.9 g/cm$^3$) and Young's modulus (41-45 GPa) that are close to those of human bone (1.8-2.1 g/cm$^3$, 3-20 GPa), implying some suitable properties for physiological applications.

However, for medical implant applications magnesium-based alloys have low strength and low plasticity due to the hexagonal closed packed (h.c.p.) crystal structure of magnesium-matrix. In addition, magnesium has a low resistance to corrosion because of its strong chemical activity. Thus, the only way to use magnesium as structure materials for biodegradable endoprosthesises is to create magnesium-based alloys with improved combination of mechanical and corrosion properties.

According to ISO 3116:2007 [11] and BS EN 1753:1997 [12], the main alloying elements for industrial magnesium are the following: aluminum (Al), zinc (Zn), manganese (Mn), Silicon (Si), Rare Earth elements (RE), Zirconium (Zr), Silver (Ag), and Yttrium (Y). According to specification ASTM for magnesium alloys [13], the following alloying elements (Al, Ag, Bi, Cu, Cd, Cr, Ca, Fe, Li, Mn, Ni, Pb, RE, Sb, Si, Sn, Sr, Th, Y, Zn, Zr) have been specified for a production of magnesium alloys. Many magnesium-based alloys were developed for last decades for different field of application, some of them - for medical applications, but more often as creep resistant alloys for industrial applications. The basic grades of magnesium-alloys and their modification for different purposes are described in detail in previous applications [14], [40]. A method for the preparation of high purity magnesium is mentioned in US 5,698,158 [37].

Initially, most investigators of biodegradable endoprosthesises have selected industrial magnesium-based alloys as structural materials for endoprosthesises: AE21 [15], AZ21 [16], AZ31 [17, 18], AZ63 [19], AZ91 [18, 20], AZ91 D [21], LAE442 [18, 21] and alloy WE43 [18, 22-25]. However, attempts to use industrial magnesium alloys - even with improved properties, such as AZ91 D or WE43 - for developing qualitative biosoluble endoprosthesises have not given expected results. During tests *in vivo,* the short time taken for full dissolution of stents made even of the best alloys appears insufficient. For example, in the case of alloy AE21 (domestic pigs test) it was less than 60 days [11], for alloy WE43 (Biotronik AMS stent, human coronary trial) it was much less than 4 months [26, 27]. An initial inspection using intravascular ultrasound (IVUS), carried out after 4 months, found no traces of stent material. It is clear that both types of stents lost mechanical integrity much earlier than the time taken for full dissolution, in spite of the fact that strut thicknesses were increased up to 150 - 200 microns [11] to compensate for insufficient mechanical properties of alloys and high corrosion rate.

As a consequence of increased thickness, the flexibility of stents with such geometry decreased, and such devices were found difficult to deliver through the vessels system. Therefore, catheters of wider diameters (6F) and predilatation of a vessel were used [28] in order to introduce the endoprosthesis into the damaged segment of a vessel. In addition, more pressure in balloon catheter was necessary to expand stent to the necessary diameter. Moreover, the early loss of a stent mechanical integrity most likely resulted in secondary vessel occlusion by flaps, spasm or thrombosis, and human trials with the Biotronik AMS stent were stopped because the degradation process compromised the scaffolding integrity [29].

So, regardless of a potentiality of magnesium alloys using in the field of biocorrodible endoprosthesises, magnesium-based alloys that were tested in trials can be used only to a limited extent because of their poor corrosion resistance and mechanical properties. For achievement of properties that will be more suitable for constructional materials of biodegradable stents, some new non-commercial alloys based on magnesium were developed: Mg-Mn-Zn [30], Mg-Ca [31, 32], Mg-Sc-Y-RE-Zr [14], Mg-In-Sc-Y-RE-Zr [33], Mg-Li-Al-Y-RE [34] and others. No data is available to date about the successful application of these alloys as a constructional material of endoprosthesises. Apparently, these alloys have not also provided characteristics that are sufficient for successful performance by biosoluble stents their main medical function: to prevent repeated blocking (restenosis) of coronary vessel lumen after operation PTCA with a stenting.

An analysis of existing data shows that modern magnesium-based alloys have considerably different set of mechanical and corrosion properties. Some of them have higher strength and low-ductility, while others are less strong and a little more deformable. Nevertheless, even the peak-values one of mechanical properties (e.g. Yield stress (YS), ultimate tensile stress (UTS) and, especially, elongation up to rupture ($\delta$)) for the best of known magnesium-based alloys, which are considered as potential material for endoprosthesises, are far lower than those for stainless steel 316LVM (YS - 280 MPa, UTS - 400 MPa, $\delta \sim 40$ %) that is one of widespread constructional materials of permanent stents. For example, on different data, extruded alloy AZ31, alloy LAE442, extruded alloy WE43 have elongation up to rupture about of 15 %, 18 % and 17 %, respectively, at a level of YS over the ranges of 150-200 MPa and UTS of 250-270 MPa. Our researches of stent models made of alloys that we have developed [14, 33] and follow-up calculations have shown that construction material of stents based on magnesium alloy should have elongation up to rupture better than 23% and strength properties on a levels: YS >140 MPa and UTS >170 MPa.

It is known that alloying elements, their distribution as well as the composition of the chemical compounds that they form influences the resistance to corrosion of alloy. The corrosion rate of magnesium alloys depends also on a structural condition of alloy and methods of manufacturing it.

It is difficult to compare existing data on a corrosion rate of various magnesium alloys, even received in the identical type of tests (for example, seawater immersion test), because different methods calculation of a corrosion rate (a loss of weight, a hydrogen evolution etc.) have various measurement errors. Even the same author's data about a corrosion rate for the same alloys can differ. Witte [21] gives named data for alloys LAE442 and AZ91D. The measured corrosion rates were for these alloys, respectively: 6.9 mm/years and 2.8 mm/years (in electrochemical test) and 5.535 mm/years and - 0.267 mm/years (in immersion test). However, in any case, corrosion properties of modern magnesium-based alloys wish to be the better. At the same times, in our opinion, the necessary corrosion rate should be about of 0.05 mm/years (~0.025 mg/cm$^2$/day, at the specific density of Mg alloy =1.8 g/cm$^3$), if to assume that 100-mkm stent's strut should be dissolved in about 6 months.

Thus, properties of existing magnesium-based alloys, especially plasticity and resistance to corrosion *in vivo,* are poor for constructional material of biodegradable endoprosthesises, in particular, vessel stents.

So, it is desirable, for example, to develop magnesium-based alloys having yield stress at room temperature that is more than 140 MPa, ultimate tensile strength of more than 170 MPa, elongation up to rupture more than 23% and corrosion resistance in a simulated body fluid (SBF) better than 0.025 mg/cm$^2$/day. Besides, such alloys may not comprise harmful for living body impurities (such as Ag, Al, As, Be, Cd, Cr, Hg, Sr, Th, Zn etc.) in a concentration above than 0.0001 % by weight.

There is a need in the art for a magnesium alloy having parameters that will provide a biodegradable endoprosthesis that can perform its medical function efficiently for the duration of and within its expected lifespan. For example, medical stent may dissolve *in vivo* with such an even corrosion rate that will maintain the requisite scaffolding capability over a period of time, which is necessary for treatment, and without premature mechanical failure due to loss of strength due to a decreased-thickness of strut.

## THE SUMMARY OF THE INVENTION

The present invention provides a medical device, in particular, a biodegradable endoprosthesis body such as a vessel stent, formed at least partly from a constructional material comprising super-pure magnesium, or an alloy thereof further comprising one or more (other) super-pure alloying elements. The device of the invention being formed from the said constructional material has excellent formability at room temperature, an optimal combination of strength, plasticity and corrosion resistance *in vivo* in the comparison with endoprosthesises formed from known magnesium-based alloys. The high formability facilitates manufacture of the endoprosthesis body by usual methods of metal processing: extrusion, forging, rolling, drawing, machining job etc.

The invention provides a medical biodegradable endoprosthesis body formed at least partly from a constructional material comprising an alloy of super-pure magnesium and one or more super-pure alloying elements as defined in independent claim 1. According to one embodiment, the invention provides a biodegradable endoprosthesis body formed at least partly from a constructional material consisting of super-pure magnesium, or from a constructional material consisting of an alloy of super-pure magnesium and one or more super-pure alloying elements. The limitations described throughout the application also apply to the aforementioned embodiment.

The endoprosthesis body is formed at least party from the constructional material; according to one embodiment, it is formed mostly, essentially or entirely therefrom.

The super-pure magnesium as used in the present invention has a purity of not less than 99.998 % (w/w). The super-pure magnesium contains a controlled content of each impurity in the group of iron, cobalt, nickel and copper equal to or less than 0.0002 % (w/w), preferably between 0.0002 % and 0 % (w/w), more preferably between 0.0002 % and 0.000002 % (w/w). In other words, the impurity of super-pure magnesium contains 0.0002 % (w/w) or less iron, preferably between 0.0002 % and 0 % (w/w), more preferably between 0.0002 % and 0.000002 % (w/w) iron; 0.0002 % (w/w) or less cobalt, preferably between 0.0002 % and 0 % (w/w), more preferably between 0.0002 % and 0.000002 % (w/w) cobalt; 0.0002 % (w/w) or less nickel, preferably between 0.0002 % and 0 % (w/w), more preferably between 0.0002 % and 0.000002 % (w/w) nickel; and 0.0002 % (w/w) or less copper, preferably between 0.0002 % and 0 % (w/w), more preferably between 0.0002 % and 0.000002 % (w/w) copper. The level of purity or impurity (%, w/w) is expressed as a percentage weight of the super-pure magnesium. The iron, cobalt, nickel and copper as used herein refer to the metal element.

The super-pure alloying element as used in the magnesium alloy described herein has a purity of not less than 99.99 % (w/w). The super-pure alloying element as used in the magnesium alloy of the described herein has a content of each impurity in the group iron, cobalt, nickel and copper of not more than 0.00025 % (w/w), preferably between 0.00025 % and 0 % (w/w), preferably between 0.0002 % and 0 % (w/w), more preferably between 0.00025 % and 0.00002 % (w/w).

In other words, the super-pure alloying element contains, as impurities, not more than 0.00025 % (w/w) iron, preferably between 0.00025 % and 0 % (w/w), preferably between 0.0002 % and 0 % (w/w), more preferably between 0.00025 % and 0.00002 % (w/w) iron; 0.0002 % (w/w) or less cobalt, preferably between 0.00025 % and 0 % (w/w), more preferably between 0.0002 % and 0.00002 % (w/w) cobalt; not more than 0.00025 % (w/w) nickel, preferably between 0.00025 % and 0 % (w/w), more preferably between 0.00025 % and 0.00002 % (w/w) nickel; and not more than 0.00025 % (w/w) copper, preferably between 0.00025 % and 0 % (w/w), more preferably between 0.00025 % and 0.00002 % (w/w) copper. The level of purity (%, w/w) is expressed as a percentage weight of the super-pure alloying element.

When purity or impurity is mentioned, only metal components are considered, *i.e.* non-metallic constituents such as oxygen, hydrogen nitrogen etc, are not considered.

The one or more super-pure alloying elements is chosen from indium, scandium, yttrium, gallium and rare earth elements (RE). Where more than one super-pure alloying element is present, two or more may be different REs.

Super-pure scandium as the sole or one of several (*i.e.* two or more) super-pure alloying elements may be present in a quantity of 0.1 to 15 % (w/w alloy).

Super-pure yttrium as the sole or one of several (*i.e.* two or more) super-pure alloying elements may be present in a quantity of 0.1 to 5 % (w/w alloy).

Super-pure gallium as the sole or one of several (*i.e.* two or more) super-pure alloying elements may be present in a quantity of 0.1 to 5 % (w/w alloy).

Super-pure indium as the sole or one of several (*i.e.* two or more) super-pure alloying elements may be present in a quantity of 0.1 to 5 % (w/w alloy).

A super-pure rare earth element as the sole or one of several (*i.e.* two or more) super-pure alloying elements may be present in a quantity of 0.1 to 5 % (w/w alloy). Where there is more than one rare earth element, the total of rare earth elements present may be in a quantity of 0.1 to 5 % (w/w alloy).

According to another embodiment, the invention provides an endoprosthesis body formed at least partly from the constructional material defined herein.

The present invention also relates to a biodegradable endoprosthesis body such as a screw, bolt, plate, staple, tubular mesh, stent, spiral, coil, wire, marker and catheter formed at least partly from the constructional material of the invention.

The present invention also relates to a use of a constructional material according to the invention for the manufacture of a biodegradable endoprosthesis such as a screw, bolt, plate, staple, tubular mesh, stent, spiral, wire, coil, marker and catheter. Such devices are commonly known as an endoprothesis body or implant.

## DETAILED DESCRIPTION OF THE INVENTION

Unless defined otherwise, all technical and scientific terms used herein have the same meaning as is commonly understood by one of skill in the art. The designation of numerical ranges by endpoints includes all integer numbers and, where appropriate, fractions subsumed within that range (*e.g.* 1 to 5 can include 1, 2, 3, 4 when referring to, for example, a number of items, and can also include 1.5, 2, 2.75 and 3.80, when referring to, for example, concentration). The designation of end points also includes the end point values themselves (*e.g.* from 1.0 to 5.0 includes both 1.0 and 5.0). Unless otherwise stated, all percentages, when expressing a quantity, are weight percentages. Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art.

The present invention relates to a finding by the inventors that a constructional material for a biodegradable endoprosthesis comprising super-pure magnesium or an alloy comprising super-pure magnesium and one or more super-pure alloying elements provides requisite properties such as yield stress, tensile strength, elongation up to rupture at a level that ensures an endoprosthesis formed therefrom is capable of maintaining its medical function for the duration of its expected life span.

Biodegradability degree of the endoprosthesis is determined by the rate of corrosion *in vivo* of the constructional material. The inventors have found out that the very weak or absent dependence of corrosion rate of magnesium on iron concentration over the range below 0.001% that has been stated in the art does not answer to validity. The inventors have found that, contrary to understanding of the art, further increase of magnesium purity from 99.99 % (high pure) to 99.998 % (super-pure), when there is a simultaneous decrease of iron, nickel and copper content in magnesium far lower than 0.001 %, results in an additional reduction of the corrosion rate in an aqueous solution of sodium chloride by three-four times. Besides, corrosion of super-pure material is homogenous throughout its surface and a pitting corrosion is absent.

Due to absence of pitting corrosion, a biodegradable endoprosthesis body such as a stent formed from the super-pure constructional material corrodes more evenly, maintaining its integrity for the full duration of treatment. Restenosis and inflammation are decreased, because the formation of large stent fragments - released when localized corrosion breaks up the stent into large sections still mainly uncorroded - is avoided. As a consequence of homogenous corrosion, strut thickness can be reduced, for example, from 170 microns used in the art, for example, to, for example, 90 microns, without a risk of premature loss of stent integrity. In the art, a reduction in corrosion is typically achieved using a hydrophobic coating, which adds to the costs of stent manufacture, and requires compatibility with any additional (e.g. drug) coating.

Concomitantly, the period to the full dissolution of endoprosthesis is increased three-four fold, and a quantity of evolved hydrogen per time unit is reduced also. This favorably affects a reaction of a living body towards endoprosthesis introduction.

Moreover, such low levels of undesirable impurities strongly change not only corrosion rate and a degree of homogeneity of corrosion, but even the composition of corrosion product of the explored magnesium materials is changed: instead of usual floccus products of dissociation (hydroxides, chlorides) the inventors observed a firm surface layer. This layer is protective and additionally lowers corrosion rate. The X-ray diffraction analysis has shown presence in this layer of the new compound, which was not observed earlier in earlier studies of corrosion of magnesium materials. This new compound has rhombic lattice having parameters a=5.864A, b=2.353A, c =4.206 A.

One embodiment of the invention provides a medical biodegradable endoprosthesis body, formed at least partly from a constructional material comprising an alloy of super-pure magnesium and one or more super-pure alloying elements as defined in independent claim 1.

Another embodiment of the invention provides a method for manufacture of a constructional material for a medical biodegradable endoprosthesis body, comprising the step of combining super-pure magnesium and one or more super-pure alloying elements to form an alloy.

The super-pure magnesium as used in the present invention has a purity of not less than 99.998 % (w/w). The purity refers to quantity of magnesium compared with the total metal content of the super-pure magnesium. The super-pure magnesium has a controlled content of each impurity in the group of iron, cobalt, nickel and copper, equal to or less than 0.0002% (w/w), preferably between 0.0002 % and 0 % (w/w), more preferably between 0.0002 % and 0.000002 % (w/w). In other words, the super-pure magnesium contains, as impurities, 0.0002 % (w/w) or less iron, preferably between 0.0002 % and 0 % (w/w), more preferably between 0.0002 % and 0.000002 % (w/w) iron; 0.0002 % (w/w) or less cobalt, preferably between 0.0002 % and 0 % (w/w), more preferably between 0.0002 % and 0.000002 % (w/w) cobalt; 0.0002 % (w/w) or less nickel, preferably between 0.0002 % and 0 % (w/w), more preferably between 0.0002 % and 0.000002 % (w/w) nickel; and 0.0002 % (w/w) or less copper, preferably between 0.0002 % and 0 % (w/w), more preferably between 0.0002 % and 0.000002 % (w/w) copper. Besides, such super-pure magnesium may not comprise impurities harmful for the living (*e.g.* human or animal) body such as Ag, Al, As, Be, Cd, Cr, Hg, Sr, Th, Zn etc. in a concentration above than 0.0001 % (w/w). The impurity refers to the quantity of metal impurity compared with the total metal content of the super-pure magnesium. Preferably, the super-pure magnesium has both the above-specified purity and impurity levels.

Each and every super-pure alloying element present in the alloy has a purity of not less than 99.99 % (w/w). The purity refers to the quantity of alloying element compared with the total metal content of the super-pure alloying element. Each and every super-pure alloying element has a content of each impurity from the group iron, cobalt, nickel and copper, of not more than 0.00025 % (w/w), preferably between 0.00025 % and 0.00002 % (w/w).

In other words, the impurity in the super-pure alloying element comprises not more than 0.00025 % (w/w) iron, preferably between 0.00025 % and 0 % (w/w), preferably between 0.0002 % and 0 % (w/w), more preferably between 0.00025 % and 0.00002 % (w/w) iron, 0.0002 % (w/w) or less; cobalt, preferably between 0.00025 % and 0 % (w/w), preferably between 0.0002 % and 0 % (w/w), more preferably between 0.0002 % and 0.00002 % (w/w) cobalt; not more than 0.00025 % (w/w) nickel, preferably between 0.00025 % and 0 % (w/w), more preferably between 0.00025 % and 0.00002 % (w/w) nickel and not more than 0.00025 % (w/w); copper; preferably between 0.00025 % and 0 % (w/w), more preferably between 0.00025 % and 0.00002 % (w/w) copper. The impurity refers to the quantity of metal impurity compared with the total metal content of the super-pure alloying element in question. Thus, when purity or impurity is mentioned, only metals are considered; non-metallic constituents such as oxygen, hydrogen, nitrogen etc, are not considered. Besides, each super-pure alloying element may not comprise impurities harmful for the living (*e.g.* human or animal) body such as Ag, Al, As, Be, Cd, Cr, Hg, Sr, Th, Zn etc. in a concentration above than 0.0005 % (w/w). The impurity refers to the quantity of metal impurity compared with the total metal content of the super-pure alloying element. Preferably, the super-pure magnesium has both the above-specified purity and impurity levels.

In another embodiment, the invention provides a biodegradable endoprosthesis formed from a constructional material comprising an alloy of super-pure magnesium and one or more super-pure alloying elements, wherein the one or more super-pure alloying elements is chosen from indium, scandium, yttrium, gallium or rare earth elements (RE). In another embodiment, the invention provides a method for the manufacture of the constructional material for a biodegradable endoprosthesis, wherein the one or more super-pure alloying elements is chosen from indium, scandium, yttrium, gallium and rare earth elements (RE). Where more than one super-pure alloying element is present, two or more may be REs. The number of super-pure alloying elements in the alloy may be 1, 2, 3, 4, 5, 6 or more.

Super-pure scandium as the sole or one of several (*i.e.* two or more) super-pure alloying elements may be present in a quantity equal to 0, 0.1, 0.5, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5 13, 13.5 14, 14.5 or 15 % (w/w alloy) or a value in the range between any two of the aforementioned values, preferably between 0.1 and 15 %, more preferably between 0.1 and 5 %. According to various data, scandium has a limit of solubility in magnesium up to 28 %. The addition of scandium to magnesium within the limits up to 15 % provides creation of Mg-Sc solid solution after homogenization of the ingot. It increases plasticity and strength of the alloy and slightly increases corrosion rate in the sodium chloride solution (at scandium content more than 5 %). Scandium is also good modifier of grain structure of magnesium ingots. Scandium additions to magnesium-based alloys improve foundry characteristics, corrosion resistance and/or mechanical strengths.

Super-pure yttrium as the sole or one of several (*i.e.* two or more) super-pure alloying elements may be present in a quantity of 0, 0.1, 0.5, 1, 1.5, 2, 2.5, 3, 3.5, 4, 4.5, 5 % (w/w alloy) or a value in the range between any two of the aforementioned values. Preferably it is present in a quantity between 0.1 and 5.0 % (w/w alloy). Yttrium has the limit of solubility in magnesium of about 2 to 6 % at room temperature. The addition up to 4 % of yttrium to magnesium increases its strength without essential reduction in plasticity and in corrosion resistance of Mg-Y alloy. Yttrium may also influence the suppression of smooth muscles cell proliferation (restenosis prevention), etc, thereby providing a therapeutic function suitable for vascular prosthesis such as a stent.

Super-pure indium as the sole or one of several (*i.e.* two or more) super-pure alloying elements may be present in a quantity of 0, 0.1, 0.5, 1, 1.5, 2, 2.5, 3, 3.5, 4, 4.5, 5 % (w/w alloy) or a value in the range between any two of the aforementioned values. Preferably, it is present in a quantity between 0.1 and 5.0 % (w/w alloy). Research by the inventors on the multi-component magnesium alloys has revealed an additional benefit of super-pure indium. For instance, addition of super-pure indium to a Mg-Sc-Y-RE-Zr alloy system, leads to an abrupt grain refinement during crystallization thereof due to creation of inter-metallic phases between scandium, yttrium and indium. The semi-finished products containing indium so formed after extrusion, blacksmithing or equal-channel angular extrusion possess a unique characteristic for magnesium alloys formability. At room temperature the alloy can withstand, without fracture, deformations up to 90 % by drawing (some passes), and up to 30 % by rolling (per one pass) without intermediate annealings. Such a high deformability is so far only known for some binary alloys Mg-Li.

Corrosion test (immersion) has shown that an additional benefit of indium when added to an alloy of the system Mg-Sc-Y-RE; it leads to reduction of the corrosion rate.

As far as medical applications are concerned, the present alloys may be used safely, for example in implants such as stents or staples. Data regarding toxicity and common influence of indium chemical compounds on humans indicate it is safe. Indium is included in the FDA's GRAS list (Generally Recognized as Safe).

According to one embodiment of the invention, indium can be replaced in the same quantity (w/w) with gallium that offers similar influence on properties in the alloy. Alternatively, alloying of magnesium with indium and gallium is also possible.

Super-pure gallium as the sole or one of several (*i.e.* two or more) super-pure alloying elements may be present in a quantity of 0, 0.1, 0.5, 1.0, 1.5, 2.0, 2.5, 3.0, 3.5, 4.0, 4.5, 5.0 % (w/w alloy) or a value in the range between any two of the aforementioned values. Preferably it is between 0.1 and 5.0 % (w/w alloy). Indium can be replaced in the same quantity (w/w) with gallium that offers similar influence on properties in the alloy. Alternatively, alloying of magnesium with a mixture of indium and gallium is also within the scope of the invention, in which case, the indium and gallium may be present in a quantity of 0, 0.1, 0.5, 1.0, 1.5, 2.0, 2.5, 3, 3.5, 4.0, 4.5, 5.0 % (w/w alloy) or a value in the range between any two of the aforementioned values. Preferably, it is between 0.1 and 5.0 % (w/w alloy).

A super-pure rare earth element (RE) as the sole or one of several (*i.e.* two or more) super-pure alloying elements may be present in a quantity of 0, 0.1, 0.5, 1.0, 1.5, 2.0, 2.5, 3.0, 3.5, 4.0, 4.5, 5.0 % (w/w alloy) or a value in the range between any two of the aforementioned values. Preferably it is between 0.1 and 5.0 % (w/w alloy). Where there is more than one rare earth element, the total of rare earth elements present may be in a quantity 0, 0.1, 0.5, 1.0, 1.5, 2.0, 2.5, 3.0, 3.5, 4.0, 4.5, 5.0 % (w/w alloy) or a value in the range between any two of the aforementioned values, preferably between 0 and 5.0 % (w/w alloy). The RE is preferable chosen from the Lanthanide Series (*i.e.* Lanthanum (La), Cerium (Ce), Praseodymium (Pr), Neodymium (Nd), Promethium (Pm), Samarium (Sm), Europium (Eu), Gadolinium (Gd), Terbium (Tb), Dysprosium (Dy), Holmium (Ho), Erbium (Er), Thulium (Tm), Ytterbium (Yb) or Lutetium (Lu)). The influence of rare earth elements on properties of magnesium alloys depends on their solubility in magnesium alloys and their melting point. Solubility of RE in solid magnesium ranges from practically zero (La) up to 7 percent (Lu). Metals from group with nuclear numbers from 64 (Gd) up to 71 (Lu) have melting temperatures and limits of solubility in magnesium higher than metals of cerium group. Alloying up to 5 % RE with magnesium raises strength and corrosion resistance of alloy. Besides, rare earth metals reduce micro-porosity of magnesium alloys during production of an initial ingot.

The concentration range of each super-pure alloying element has been specified over the above range of concentrations, which optionally includes 0 %. This designates that the indicated alloying element may be absent from the material so formed. In the case of a single-component material, the proposed constructional material would contain only super-pure magnesium.

The alloy contained in the endoprosthesis has an improved combination of strength, plasticity and high corrosion resistance in body liquids, high formability at ambient temperature in comparison with existing magnesium alloys. The high formability allows certain forms to be made by usual methods of metals processing - extrusion, forging, rolling, drawing, machining job etc.

The constructional material may comprise super-pure magnesium (Mg) or an alloy thereof with one or more super-pure alloying elements (Sc, Y, In, Ga, RE) in the following combinations:

- Single-component material: super-pure magnesium.

- Two-component alloy: Mg-Sc, Mg-Y, Mg-In, Mg-Ga, or Mg-RE.

- Three-component alloy: Mg-Sc-Y, Mg-Sc-In, Mg-Sc-Ga, Mg-Sc-RE, Mg-Y-In, Mg-Y-Ga, Mg-Y-RE, Mg-In-Ga, Mg-In-RE, or Mg-Ga-RE.

- Four-component alloy: Mg-Sc-Y-In, Mg-Sc-Y-Ga, Mg-Sc-Y-Re, Mg-Sc-In-Ga, Mg-Sc-In-RE, Mg-Sc-Ga-RE, Mg-Y-In-Ga, Mg-Y-In-RE, Mg-Y-Ga-RE, or Mg-In-Ga-RE.

- Five-component alloy: Mg-Sc-Y-In-Ga, Mg-Sc-Y-In-RE, Mg-Sc-Y-Ga-RE, Mg-Sc-In-Ga-RE, or Mg-Y-In-Ga-RE.

- Six-component alloy: Mg-Sc-Y-In-Ga-RE.

According to one aspect of the invention, biodegradable endoprosthesis body is at least partly formed from a constructional material comprising super-pure magnesium. According to another aspect of the invention, biodegradable endoprosthesis body is at least partly formed from a constructional material comprising an alloy of Mg-Sc, Mg-Y, Mg-Sc-In, Mg-Sc-Y, Mg-Sc-Y-In, or Mg-Sc-Y-In-RE.

A general increase of magnesium purity (and of the alloying elements) results in improvement of plastic properties e.g. elongation up to rupture, formability, and in some reduction in strength properties (YS, UTS). The strength and plasticity of a metal sharply rises, when a grain size of the metal is reduced. The relationship between the flow stress ($\sigma$) and the grain size (d) is defined by the equation of Hall - Petch - Stroh:

$$\sigma = \sigma\,(0) + k\,/\,\sqrt{d}, \qquad\qquad (1)$$

(wherein, $\sigma$ (0) and k are constant).

Other things being equal, the strength of a metal material increases in inverse proportion to the square root of the grain size. There is not strong dependence of plasticity on grain size of metal, but it is the fact that it increases with decreasing of grain size. An increasing ratio depends on operating mechanism of plastic deformation.

Usual (industrial) methods of deformation processing of metals allow a grain size not less than 10-20 micrometers to be achieved; this may not be sufficient for essential increase of their strength and plastic characteristics. It is known that metallic materials with the ultra fine-grained structure (UFG) show higher level of mechanical characteristics and have higher deformability. However, it is often difficult to create such structures in materials having low plasticity in initial conditions (for example, in ingots).

According to one embodiment of the invention, the biodegradable endoprosthesis is at least partly formed from a constructional material, that has the grain size of less than 5 microns and comprising super-pure magnesium or alloy of super-pure magnesium and super-pure alloying elements,.

The present inventors have found that the UFG structure with the grain size of 0.1 - 3.0 microns can be achieved by a method of intensive deformation that comprises repeated alternation of a straight-through extrusion and a settlement (it gives a high component of shear stress during a deformation) in a complex with the programmed heat treatment for such non-conventional materials as beryllium and niobium-titanium super-conducting alloys. The inventors found that the strength is increased by 30 % and plasticity many times [35]. It is also possible to use an intensive deformation, *i.e.* changing of materials flow direction for the creation of shear stress, during the processing of materials. Then the developed method of intensive deformation has been applied to magnesium and its alloys.

For additional improvement in the combination of mechanical and corrosion characteristics of the present alloys, the alloy of invention may be used in ultra fine-grained (UFG) condition with a grain size of 5 microns or less. The UFG structure is created in preliminary forged (extruded) ingots by methods of programmed intensive plastic deformation in combination with programmed heat treatment.

Further setting up of any necessary product form (for practical using) can be made according to any known technological schemes: rolling, extrusion, press forming etc.

The super-pure magnesium (Mg) and each super-pure alloying element (alloying element *i.e.* scandium, yttrium, indium, gallium, or RE) that were used for preparation of the constructional material of present invention have a purity much higher than that for commercially pure elements. The inventors have produced the super-pure magnesium and necessary components of the alloy thereof contained in the endoprosthesis body by a combination known methods to refine each metal, namely multi-stage vacuum distillation using a condenser with a gradient of temperature as described by Ivanov *et al* [36]. The purification method has been described in the Patent US 5 698 158 [37] provides an uncertain content of zinc in purified magnesium. We consider this element as undesirable in applications of magnesium and its alloys as constructional material of medical biosoluble endoprosthesises. Zinc is included, for example, into the first ten of heavy metals, which content is limited in foodstuff.

Content of each impurity in the constructional material of biodegradable endoprosthesis was measured by method laser mass-spectrometry (EMAL-2) with double focusing by the method Mattauch-Gerzog [38]. The sensitivity of the said instrument of high precision is about $1 \times 10^{-6}$ % w/w for any element having an atomic number of more than 3.

The alloy for the constructional material of a biodegradable endoprosthesis is prepared using the known methods for the preparation of ingot of magnesium-based alloys as described, for example, by Lipnitsky and Morozov [39]. Generally, the said alloy is prepared by the direct fusion of super-pure magnesium with the specified elements in a high-frequency induction furnace having an atmosphere of high purity argon and in a high purity graphite crucible. For full dissolution of all components, the alloy is stood in the crucible at the temperature of 700, 710, 720, 730, 740, 750, 760, 770, 780, 790, 800, 810, 820, or 830 degree Celsius or a temperature in a range between any two of the aforementioned values, preferably between 760 to 780 degree Celsius.

The super-pure magnesium or super-pure alloy thereof as defined herein is suitable for use in any biodegradable medical device, including an endoprothesis body or endoprosthesis, which has contact with a living body fluid and/or tissue *in situ.* An example of an endoprosthesis body includes a screw, bolt, plate, staple, tubular mesh, stent, spiral, coil, wire, marker or catheter. Such endoprosthesis bodies are well known in the art. Where the endoprosthesis body is a stent, for example, it may be a cylinder which that is perforated with passages that are slots, ovoid, circular, regular, irregular or the like shape. It may also be composed of helically wound or serpentine wire structure in which the spaces between the wires form the passages. A stent may also be flat perforated structure that is subsequently rolled to form a tubular structure or cylindrical structure that is woven, wrapped, drilled, etched or cut to form passages. Such cylinder or wires may be formed from the structural material defined herein. A stent may also be combined with a graft to form a composite medical device, often referred to as a stent graft. A stent may capable of being coated with a composition. The endoprosthesis body may be implantable. One embodiment of the invention is an endoprosthesis body formed at least partly from the super-pure constructional material defined herein. It will be understood that the endoprosthesis body defined herein inevitably contains the super-pure constructional material.

The inventors, on the basis of the existing references and their own research, have chosen as preferable embodiments an endoprosthesis body at least partly formed from the described constructional material that has the optimal combination of mechanical and corrosion characteristics at the room temperature (among the known magnesium-based alloys).

## SOME PREFERRED EMBODIMENTS

According to one embodiment, the invention provides a biodegradable endoprosthesis body formed at least partly from a constructional material comprising an alloy of super-pure magnesium and one or more super-pure alloying elements as defined in claim 1. According to one embodiment, the invention provides a biodegradable endoprosthesis body formed at least partly from a constructional material consisting of an alloy of super-pure magnesium and one or more super-pure alloying elements as defined in claim 1. The limitations described throughout the application also apply to the aforementioned embodiment.

According to another embodiment, the invention provides a biodegradable endoprosthesis body formed at least partly from a constructional material comprising an alloy of super-pure magnesium and further comprising one or more super-pure alloying elements, wherein said super-pure magnesium has a purity of not less than 99.998 % (w/w), and wherein the super-pure magnesium contains an impurity in the group iron, cobalt, nickel and copper in a quantity of equal to or less than 0.0002 %, preferably between 0.0002 % and 0 % (w/w), more preferably between 0.0002 % to 0.000002 % (w/w) of each said impurity.

According to another embodiment, the invention provides a biodegradable endoprosthesis body formed at least partly from a constructional material comprising an alloy of super-pure magnesium and further comprising one or more super-pure alloying elements, wherein said super-pure magnesium has a purity of not less than 99.998 % (w/w), and wherein the super-pure magnesium contains an impurity in the group iron, cobalt, nickel and copper in a quantity of equal to or less than 0.0002 %, preferably between 0.0002 % and 0 % (w/w), more preferably between 0.0002 % to 0.000002 % (w/w) of each said impurity.

Each and every super-pure alloying element forming the aforementioned alloys has a purity of not less than 99.99 % (w/w) and contains an impurity in the group iron, cobalt, nickel and copper in a quantity of no more than 0.00025 % (w/w), preferably between 0.00025 % and 0 % (w/w), more preferably between 0.00025 % and 0.00002 % (w/w) of each said impurity.

According to another embodiment, the invention provides a medical biodegradable endoprosthesis body, formed at least partly from a constructional material comprising an alloy of super-pure magnesium, further comprising one or more super-pure alloying elements, wherein

- the super-pure magnesium has a purity of not less than 99.998 % (w/w) and contains a level of impurity of iron, cobalt, nickel and copper, each equal to or less than 0.0002 % (w/w) of each said impurity;

- one or more super-pure alloying elements each has a purity not lower than 99.99 % (w/w) and each contains impurity of iron, cobalt, nickel and copper at a level of no more than 0.00025 % (w/w) of each said impurity.

According to another embodiment, the invention provides the biodegradable endoprosthesis body formed at least partly from a constructional material comprising an alloy of super-pure magnesium and one or more super-pure alloying elements, wherein the one or more super-pure alloying elements are chosen from indium, scandium, yttrium, gallium and one or more rare earth elements (RE).

According to another embodiment, the invention provides the biodegradable endoprosthesis body formed at least partly from a constructional material comprising an alloy of super-pure magnesium and super-pure scandium, wherein the content of super-pure scandium in the alloy is between 0.1 to 15 % (w/w).

According to another embodiment, the invention provides the biodegradable endoprosthesis body formed at least partly from a constructional material comprising an alloy of super-pure magnesium and super-pure yttrium, wherein the content of super-pure yttrium in the alloy is between 0.1 and 5 % (w/w).

According to another embodiment, the invention provides the biodegradable endoprosthesis body formed at least partly from a constructional material comprising an alloy of super-pure magnesium and super-pure indium, wherein the content of super-pure indium in the alloy is between 0.1 and 5 % (w/w).

According to another embodiment, the invention provides the biodegradable endoprosthesis body formed at least partly from a constructional material comprising an alloy of super-pure magnesium and super-pure gallium, wherein the content of super-pure gallium in the alloy is between 0.1 and 5 % (w/w).

According to another embodiment, the invention provides the biodegradable endoprosthesis body formed at least partly from a constructional material comprising an alloy of super-pure magnesium and super-pure gallium and super-pure indium, wherein the content of super-pure gallium and super pure indium combined in the alloy is between 0.1 and 5 % (w/w).

According to another embodiment, the invention provides the biodegradable endoprosthesis body formed at least partly from a constructional material comprising an alloy of super-pure magnesium and one or more super-pure rare earth elements (RE), wherein the content of super-pure rare earth elements (RE) in the alloy is between 0.1 and 5 % (w/w).

According to another embodiment, the invention provides the biodegradable endoprosthesis body as described above, wherein the constructional material has a grain size of less than 5 microns.

According to another embodiment, the invention provides medical biodegradable endoprosthesis body, formed at least partly from the constructional material as defined herein.

According to another embodiment, the invention provides the biodegradable endoprosthesis body, wherein the named endoprosthesis is medical stent. Another embodiment of the invention is a medical stent formed at least partly from the constructional material as defined herein.

According to another embodiment, the invention provides the biodegradable endoprosthesis body, wherein the named medical stent is a platform for drug-eluting stent. Another embodiment of the invention is a drug-eluting medical stent formed at least partly from the constructional material as defined herein.

According to another embodiment, the invention provides the biodegradable endoprosthesis body, wherein the named endoprosthesis is a medical staple. Another embodiment of the invention is a medical staple formed at least partly from the constructional material as defined herein.

According to another embodiment, the invention provides the biodegradable endoprosthesis body, wherein the named endoprosthesis is medical bolt. Another embodiment of the invention is a medical bolt formed at least partly from the constructional material as defined herein.

According to another embodiment, the invention provides the biodegradable endoprosthesis body, wherein the named endoprosthesis is medical plate. Another embodiment of the invention is a medical plate formed at least partly from the constructional material as defined herein.

According to another embodiment, the invention provides the biodegradable endoprosthesis body, wherein the named endoprosthesis is medical coil. Another embodiment of the invention is a medical coil formed at least partly from the constructional material as defined herein.

According to another embodiment, the invention provides the biodegradable endoprosthesis body, wherein the named endoprosthesis is medical X-ray marker. Another embodiment of the invention is a medical X-ray marker formed at least partly from the constructional material as defined herein.

According to another embodiment, the invention provides the biodegradable endoprosthesis body, wherein the named endoprosthesis is medical catheter. Another embodiment of the invention is a medical catheter formed at least partly from the constructional material of the invention. Another embodiment of the invention is a medical catheter formed from or comprising the construction material of the invention.

According to another embodiment, the invention provides the biodegradable endoprosthesis body, wherein the named endoprosthesis is medical screw, tubular mesh, wire or spiral. Another embodiment of the invention is a medical screw, tubular mesh, wire or spiral formed at least partly from the constructional material of the invention.

According to another embodiment, the invention provides a use of a constructional material as defined herein, for the manufacture of an endoprosthesis body as defined herein.

According to another embodiment, the invention provides the biodegradable endoprosthesis body, wherein the named super-pure constructional material is, at least, a part of biocorrodible endoprosthesis body. Another embodiment of the invention is a biodegradable endoprosthesis body formed at least partly from the super-pure constructional material of the invention.

The endoprosthesis body described is formed at least party from the constructional material; according to one embodiment, it may be formed mostly, essentially or entirely therefrom.

Another embodiment of the invention is a use of the constructional material as defined herein, for the manufacture of an endoprosthesis body as defined herein.

Another embodiment of the invention is a constructional material as defined herein i.e. comprising super-pure magnesium, or comprising an alloy of super-pure magnesium and one or more super-pure alloying elements.

## EXAMPLES

### Example 1

On available data, researchers have distinguished three grades of magnesium: low pure (LP) (~99.9 % Mg), commercially pure (CP) (~99.95 % Mg) and high pure (HP) (~99.98 % Mg). Content of iron, copper and nickel is restricted by the following limits (Table 1):

Table 1. The terminal concentrations of iron, copper, cobalt and nickel for different grades of magnesium.

| Grade of Mg | Total purity of Mg, % wt. | Content, % wt. | | | |
|---|---|---|---|---|---|
| | | Fe | Co | Cu | Ni |
| LP (low pure) | ~ 99.9 | 0.028 | - | 0.001 | 0.002 |
| CP (commercial pure or pure) | ~ 99.95 | 0.020 | - | 0.002 | 0.002 |
| HP (high pure) | ~ 99.98 | 0.004 | - | 0.002 | 0.0009 |
| SP (super pure; used in the invention) | > 99.998 | < 0.0002 | < 0.00002 | < 0.0002 | < 0.0002 |

We have carried out a purification of magnesium up to the purity of 99.998 % and more (only metal impurities are taken into consideration) by the way of double (triple) distillation in ultra-high vacuum. Such processing of commercially pure magnesium has resulted in decrease concentrations of adverse for corrosion resistance impurities (iron, nickel and copper) to a level equal to or below of 0.0002 % by weight and less for each specified impurity. Content of cobalt was reduced down to 0.00002 % w/w.

The specified content of iron, nickel and copper has resulted in essential improving of corrosion resistance of super-pure (SP) magnesium in comparison with CP and HP magnesium. The measured corrosion rates of the named grades of magnesium (immersion test, 0.9 % water solution NaCl, the method of weight loss measurement) were: CP Mg - 50 mg/cm$^2$/day, HP Mg - 2 mg/cm$^2$/day, SP Mg (used in the invention) - less than 0.01 mg/cm$^2$/day.

The further purification of magnesium up to iron, cobalt, nickel and copper content down to a level about 0.00002 % (w/w) led to additional lowering of corrosion rate, and the obtained level (less that 0.005 mg/cm$^2$/day) for such material is lower than that is necessary for practical purposes. Moreover, when the levels of undesirable impurities are 0.0002 % (w/w) and lower, they strongly change not only corrosion rate and a degree of homogeneity of corrosion, but even the composition of corrosion product of the explored magnesium materials is changed: instead of usual floccus products of dissociation (hydrochlorides) we observed the firm layer on their surface. This layer is protective and additionally lowers corrosion rate. The X-ray diffraction analysis has shown presence in this layer of the new compound, which was not observed in earlier studies of corrosion of magnesium materials. This new compound has rhombic lattice having parameters **a**=5.864A, **b**=2.353A, **c** =4.206 A. The preferred concentrations of iron, cobalt, nickel and copper for magnesium and its alloys may be on the level of about 0.0002 to 0.000002 % (w/w) each by weight. This will ensure an optimal corrosion rate of biodegradable endoprosthesises and necessary uniformity of corrosion process. At the same time, uniformity of corrosion may be a relevant parameter too, because, even at low common level of corrosion rate, overetching (due to a pitting corrosion) of some struts, for example, leads to a loss of stent integrity and of possibility to provide scaffold function.

To keep a low content of undesirable impurities in the alloy comprising super-pure magnesium described herein, the inventors have used for the alloy material, alloying components that were also super-pure (99.99 % w/w or better). They have prepared necessary alloying elements containing in each of them no more than 0.00025 % of each impurity in the group: iron, cobalt, nickel and copper.

In spite of the fact that all alloying elements used by the inventors are more noble concerning magnesium (on hydrogen potential) and, therefore, would be expected to raise the corrosion rate of the alloy so formed, the inventors have found out that, contrary to expectation, an appreciable increase of corrosion rate of an alloy as described herein has not occurred: the corrosion rate was about 0.020 mg/cm$^2$/day.

The influence of alloying elements on the mechanical and corrosion properties of magnesium alloys was well studied for binary systems, but in multi-component alloy their mutual and aggregate influence can turn out to be complex and unpredictable. Therefore, the choice of the basic alloying elements and their interrelations in an alloy are the controlling factor for its future properties.

The inventors, when considering the alloying elements, discriminate the group of rare-earth elements (RE) - elements with numbers from 57 up to 71 in the Periodic table - from both yttrium and scandium. Though yttrium and scandium have an external electronic shell structure that is identical to RE, and a similarity with some of the chemical properties of RE, they would be expected to differ from RE in alloy compositions, according to ASTM standard, because they differ in an influence on alloys properties.

Basic alloying elements for the magnesium-based alloys used in the endoprosthesis body of the invention, namely; indium, gallium, scandium, yttrium and RE, provide alloys with favorable characteristics (for example, plasticity) and yet do not change essentially other characteristics (for example, resistance to corrosium). Alloys of the invention contain alloying elements in the quantities that far less their solubility in magnesium. It is desirable also not to have in an alloy composition such elements that have a negative influence on a living body. This requirement is met by the high general purity of offered alloys.

## Example 2

Ingot of super-pure magnesium (99.999 % magnesium, content of iron, copper and nickel is about of 0.00016 %, by weight, of each; content of cobalt was less than of 0.00001 % w/w) was extruded from diameter of 50 mm to diameter of 30 at the temperature of 290°C. Then the obtained semi-finished product was subjected to deformation by equal-channel angular extrusion at the temperature of 270-240°C, number of cycles of extrusion - 6, with intermediate annealing at the temperature of 280°C through 2 - 3 cycles. Samples were cut out from the obtained extrudate for the tensile test at room temperature and tests for corrosion (in 0.9 % sodium chloride water solution).

### Test results 2

Mechanical properties (after annealing at the temperature of 150°C within one hour): YS=142 MPa, UTS=165 MPa, elongation=28 %.

The corrosion rate (calculated from a weight loss of specimens and by quantitative definition of the magnesium, which has passed in the solution, through the fixed time intervals): 0.008 mg/cm$^2$/day.

Results of tests show that this material of the invention has the best of known corrosion properties in comparison with the widespread industrial alloys of magnesium.

## Example 3

An alloy contained essentially of magnesium with purity of 99.998 % with addition of (% by weight) 8 % scandium and 2.7 % yttrium. Contents iron, nickel and copper in the alloy did not exceed 0.00024 % of each, and contents of incidental elements and impurities did not exceed 0.0002 %. The alloy was made by a way of the direct fusion of magnesium with the preliminary prepared master alloy with the specified elements in a high-frequency induction furnace having an atmosphere of high purity argon and in a high purity graphite crucible.

For full dissolution of alloying components, the alloy was stood in the crucible at the temperature of 770°C within 30 minutes and then was poured out into a cooled steel mold with a special daubing by method of bottom teem.

The obtained ingot was extruded from diameter of 50 mm to diameter of 30 mm at a temperature of 360°C. Then the obtained semi-finished product was subjected to deformation by equal-channel angular extrusion at the temperature of 350-320°C, number of cycles of extrusion 8, with intermediate annealing at the temperature of 360°C through 2 - 3 cycles (at achievement of micro-hardness H $\mu$ of 90 kg/mm$^2$).

Samples were cut out from the obtained extrudate for the tensile test at room temperature and tests for corrosion (in 0.9 % sodium chloride water solution).

### Test results 3

Mechanical properties (after annealing at the temperature of 460°C within one hour): YS=150 MPa, UTS=175 MPa, elongation=23 %.

The corrosion rate (calculated as in the example 1): 0.022 mg/cm$^2$/day.

Results of tests show that this alloy has the optimal combination of mechanical and corrosion properties in comparison with the widespread industrial alloys of magnesium.

## Example 4

An alloy containing essentially magnesium with purity of 99.998 % with addition of (% by weight) 3 % scandium, 4 % yttrium and 2 % indium. Contents iron, cobalt, nickel and copper in the alloy did not exceed 0.00022 % of each, and contents of incidental elements and impurities did not exceed 0.0002 %.

Ingot was prepared as in example 2.

The obtained ingot was extruded from a diameter of 50 mm to diameter of 30 mm at a temperature of 370°C. Then the obtained semi-finished product was subjected to deformation by equal-channel angular extrusion at the temperature of 350-330°C, number of cycles of extrusion was 8, with intermediate annealing at the temperature of 360°C through 2 - 3 cycles (at achievement of micro-hardness H $\mu$ of 95 kg/mm$^2$).

Samples have been cut out from the obtained extrudate for the tensile test at room temperature and tests for corrosion.

Test results 4

Mechanical properties (after annealing at the temperature of 460°C within one hour): YS=165 MPa, UTS=195 MPa, elongation=25 %.

The corrosion rate (calculated as in the example 1): 0.02 mg/cm$^2$/day.

Results of tests show that this alloy has the optimal combination of mechanical and corrosion properties in comparison with the widespread industrial alloys of magnesium.

**REFERENCES**

[1] Bolz, Armin, Popp, Thomas. Implantable, bioresorbable vessel wall support, in particular coronary stent. US Patent 6287332, Pub. date: 09/11/2001

[2] Peuster M, Fink C, Wohlsein P, Bruegmann M, Gunther A, Kaese V, et al. Degradation of tungsten coils implanted into the subclavian artery of New Zealand white rabbits is not associated with local or systemic toxicity. Biomaterials 2003;24:393-399.

[3] M. Peuster, P. Wohlsein, M. Brügmann, M. Ehlerding, K. Seidler, C. Fink, H. Brauer, A. Fischer, G. Hausdorf. A novel approach to temporary stenting: degradable cardiovascular stents produced from corrodible metal-results 6-18 months after implantation into New Zealand white rabbits. Heart 2001; 86: p.p. 563-569.

[4] Waksman R, Pakala R, Baffour R, Seabron R, Hellinga D, Fermin O. Short-term effects of biocorrodible iron stents in porcine coronary arteries. J Interv Cardiol 2008;21:15-20.

[5] B. Heublein, G. Hausdorf. Metallic implant which is degradable in vivo. US 2002 0 004 060 A1, January 10, 2002,

[6] N. Adden, A Borck. Stent having a coating. US 2009 0 076 596, March 19, 2009.

[7] H. Mueller, J. Loeffler, P. Uggowitzer. Implant with a base body of a biocorrodible iron alloy. Patent Application, Pub. No. US 2009/0198320 A1, Aug. 6, 2009.

[8] H. Hermawan, D. Dube, D. Mantovani. Development of degradable Fe-35Mn Alloy for biomedical application. Adv Mater Res 2007; 15-17: p.p.107-112.

[9] Vormann J. Magnesium: nutrition and metabolism. Mol Aspects Med 2003;24:27-37

[10] H. Zreiqat, C.R. Howlett, A. Zannettion, P. Evans, G. Schulze-Tanzil and C. Knabe, J. Biomed. Mater. Res. 62 (2002), p. 175.

[11] ISO-3116. Magnesium and magnesium alloys - wrought magnesium alloys. International Standard Organization; 2007.

[12] BS EN 1753. Magnesium and magnesium alloys. Magnesium alloy ingots and castings. 1997.

[13] ASTM-B275. Standard practice for codification of certain nonferrous metals and alloys, cast and wrought. Annual book of ASTM standards. Philadelphia, Pennsylvania, USA: American Society for Testing and Materials; 2005.

[14] Y. Popowski, I. Papirov, A. Pikalov, S. Svitsov, V. Shokurov. Magnesium-based alloy with improved combination of mechanical and corrosion characteristics. EP 1 835 042 A1 Date of publication: 19.09.2007

[15] B. Heublein, R. Rohde, V. Kaese, M. Niemeyer, W. Hartung, A. Haverich. Biocorrosion of magnesium alloys: a new principle in cardiovascular implant technology? Heart 2003;89: p.p.651-656.

[16] Pietak A, Mahoney P, Dias GJ, Staiger MP. Bone-like matrix formation on magnesium and magnesium alloys. J Mater Sci Mater Med 2008;19(1): p.p.407-415.

[17] Muller WD, Nascimento ML, Zeddies M, Corsico M, Gassa LM, Lorenzo de Mele MAF. Magnesium and its alloys as degradable biomaterials corrosion studies using potentiodynamic and EIS electrochemical techniques. Mater Res 2007;10: p.p.5-10.

[18] Witte F, Kaese V, Switzer H, Meyer-Lindenberg A, Wirth CJ, Windhag H. In vivo corrosion of four magnesium alloys and the associated bone response. Biomaterials 2005;26: p.p.3557-3563.

[19] Liu CL, Xin YC, Tang GY, Chu PK. Influence of heat treatment on degradation behavior of biodegradable die-cast AZ63 magnesium alloy in simulated body fluid. Mater Sci Eng A 2007;456: p.p. 350-357.

[20] Kannan MB, Raman RKS. In vitro degradation and mechanical integrity of calcium-containing magnesium alloys in modified-simulated body fluid. Biomaterials 2008;29:p.p.2306-2314.

[21] Witte F, Fischer J, Nellesen J, Crostack H, Kaese V, Pischd A, et al. In vitro and in vivo corrosion measurements of magnesium alloys. Biomaterials 2006;27:p.p.1013-1018.

[22] Endoprosthesis. H. Claus; G. Bodo; H. Mueller; B. Heublein. US 2004 098108, May, 20,2004

[23] Mario C, Griffiths H, Goktekin O, Peeters N, Verbist J, Bosiers M, et al. Drugeluting bioabsorbable magnesium alloys. J Interv Cardiol 2004;17(6):391-5.

[24] Zartner P, Cesnjevar R, Singer H, Weyand M. First successful implantation of a biodegradable metal stent into the left pulmonary artery of a preterm baby. Catheter Cardiovasc Interv 2005;66:590-4.

[25] Waksman R, Pakala R, Kuchulakanti PK, Baffour R, Hellinga D, Seabron R, et al. Safety and efficacy of bioabsorbable magnesium alloy stents in porcine coronary arteries. Catheter Cardiovasc Interv 2006;68:607-17.

[26] Erbel R, Di Mario C, Bartunek J et al. Temporary scaffolding of coronary arteries with bioabsorbable magnesium stents: a prospective, non-randomised multicentre trial. Lancet (2007), 369(9576):1869-1875.

[27] R. Waksman. Biodegradable stents: They do their job and disappear. Journal of Invasive Cardiology 2006; 18(2): 70-74

[28] R. Waksman. Promise and Challenges of Bioabsorbable Stents. Catheterization and Cardiovascular Interventions 70:407-414 (2007)

[29] Biodegradable stents could be the ideal stent. Healthcare Tech Guide, Coronary Stents Portal, Author: Chris Kaiser, Sunday, 08, March, 2009.

[30] Xu L, Yu G, Zhang E, Pan F, Yang K. In vivo corrosion behavior of Mg-Mn-Zn alloy for bone implant application. J Biomed Mater Res 2007;83A(3):703-11.

[31] Li Z, Gu X, Lou S, Zheng Y. The development of binary Mg-Ca alloys for use as biodegradable materials within bone. Biomaterials 2008;29:1329-44.

[32] T. Hassel, Fr.-W. Bach, A. Golovko, C. Krause, Investigation of the mechanical properties and the corrosion behaviour of low alloyed magnesium-calcium alloys for use as absorbable biomaterial in the implant technique, in: M.O. Pekguleryuz, L.W.F., Mackenzie (Eds.), Magnesium Technology in the Global Age, 45th Annual Conference of Metallurgists of CIM, Montreal, Quebec, Canada 2006, pp. 359-370.

[33] I. Papirov, A. Pikalov, S. Svitsov, V. Shokurov. Magnesium-based alloys. EP 2 000 551, Publication Date: Dec. 12, 2008

[34] A. Meyer-Lindenberg, Andrea et al. Medical implant for the human or animal body. US Patent 2004 0241036 A1, December 2, 2004.

[35] O.V.Chernyi, N.F.Andrievskaya, V.O.Ilicheva, G.E.Storozhilov, P.J.Lee, A.A.Squitieri. The Microstructure and Critical Current Density of Nb-48wt.%Ti Superconductor with Very High Alpha-Ti Precipitate Volume and Very High Critical Current Advances in Cryogenic Engineering, 2002, vol. 48B, pp. 883-890.

[36] V.E. Ivanov, I.I. Papirov et al. Pure and high-pure metals. M. Metallurgija, 1965, 152 p.p., (in Russian).

[37] Raymond K.F. Lam. Vacuum distillation apparatus for producing ultra high purity material, US Patent 5 698 158, Publication date: Dec. 16, 1997.

[38] A.I. Boriskin, Y.S.Bruchanov at al. Mass-spectrometers with double focusing and a laser source of ions. Science Devices. 1981, No.24, p.28-30 (in Russian).

[39] A.M. Lipnitsky, I.V. Morozov. Technology of nonferrous castings. L: Mashgiz ,1986 (in Russian). 224pp.

[40] Y. Popowski, I. Papirov, A. Pikalov, S. Sivtsov, V. Shokurov. Magnesium-based alloy with improved combination of mechanical and corrosion characteristics. EP 1 835 043 A1 Date of publication: 19.09.2007

## Claims

1. A medical biodegradable endoprosthesis body, formed at least partly from a constructional material of an alloy comprising super-pure magnesium and one or more super-pure alloying elements, wherein

   - the super-pure magnesium has a purity of not less than 99.998 % (w/w) and contains a level of impurity of iron, cobalt, nickel and copper, each equal to or less than 0.0002 % (w/w) of each said impurity;
   - one or more super-pure alloying elements each has a purity not lower than 99.99 % (w/w) and each contains impurity of iron, cobalt, nickel and copper at a level of no more than 0.00025 % (w/w) of each said impurity, wherein the one or more super-pure alloying elements is chosen from scandium, yttrium, indium, gallium or one or more rare earth elements (RE).

2. The endoprosthesis body according to claim 1, wherein a content of super-pure scandium in the alloy is between 0.1 and 15 % (w/w).

3.  The endoprosthesis body according to claim 1 or 2, wherein a content of super-pure yttrium in the alloy is between 0.1 and 5 % (w/w).

4.  The endoprosthesis body according to any of claims 1 to 3, wherein a content of super-pure indium in the alloy is between 0.1 and 5 % (w/w).

5.  The endoprosthesis body according to any of claims 1 to 4, wherein a content of super-pure gallium in the alloy is between 0.1 and 5 % (w/w).

6.  The endoprosthesis body according to any of claims 1 to 5, wherein a content of one or more super-pure RE in the alloy is between 0.1 and 5% (w/w).

7.  The endoprosthesis body according to any of claims 1 to 6, wherein the constructional material has a grain size of less than 5 microns.

8.  The endoprosthesis body according to any of claims 1 to 7, which is a medical stent.

9.  The endoprosthesis body according to any of claims 1 to 7, which is a drug-eluting medical stent.

10. The endoprosthesis body according to any of claims 1 to 7, which is a medical staple.

11. The endoprosthesis body according to any of claims 1 to 7, which is a medical bolt.

12. The endoprosthesis body according to any of claims 1 to 7, which is a medical plate.

13. The endoprosthesis body according to any of claims 1 to 7, which is a medical coil.

14. The endoprosthesis body according to any of claims 1 to 7, which is an X-ray marker.

15. The endoprosthesis body according to any of claims 1 to 7, which is a medical catheter.

16. The endoprosthesis body according to any of claims 1 to 7, which is a medical screw, tubular mesh, wire or spiral.

17. Use of a constructional material as defined in any of claims 1 to 7, for the manufacture of an endoprosthesis body as defined in any of claims 8 to 16.

18. A constructional material comprising super-pure magnesium as defined in claim 1 and one or more super-pure alloying elements as defined in any of claims 1 to 7.

19. A method for the manufacture of a constructional material for a medical biodegradable endoprosthesis body, comprising the step of combining super-pure magnesium as defined in claim 1 and one or more super-pure alloying elements as defined in claim 1 to form an alloy.

20. Method according to claim 19, wherein the one or more super-pure alloying elements are combined in the alloy in the quantity defined in any of claims 2 to 6.

**Patentansprüche**

1.  Medizinischer bioabbaubarer Endoprothesekörper, wenigstens teilweise aus einem Konstruktionsmaterial aus einer Legierung gebildet, die superreines Magnesium und eine oder mehrere superreine Legierungselemente umfasst, wobei

    - das superreine Magnesium eine Reinheit von nicht weniger als 99,998 % (w/w) aufweist und eine Verunreinigungsmenge an Eisen, Kobalt, Nickel und Kupfer enthält, die für jede der Verunreinigungen jeweils kleiner oder gleich 0,0002 % (w/w) ist;

- ein oder mehrere superreine Legierungselemente jeweils eine Reinheit von nicht unter 99,99 % (w/w) aufweisen und jeweils Verunreinigungen an Eisen, Kobalt, Nickel und Kupfer in einer Menge von nicht mehr als 0,00025 % (w/w) an der jeweiligen Verunreinigung enthalten, wobei das eine oder die mehreren superreinen Legierungselemente ausgewählt ist/sind aus Scandium, Yttrium, Indium, Gallium und einem oder mehreren Seltenerdelementen (RE).

2.  Endoprothesekörper gemäß Anspruch 1, wobei der Gehalt an superreinem Scandium in der Legierung zwischen 0,1 und 15 % (w/w) beträgt.

3.  Endoprothesekörper gemäß Anspruch 1 oder 2, wobei der Gehalt an superreinem Yttrium in der Legierung zwischen 0,1 und 5 % (w/w) beträgt.

4.  Endoprothesekörper gemäß einem der Ansprüche 1 bis 3, wobei der Gehalt an superreinem Indium in der Legierung zwischen 0,1 und 5 % (w/w) beträgt.

5.  Endoprothesekörper gemäß einem der Ansprüche 1 bis 4, wobei der Gehalt an superreinem Gallium in der Legierung zwischen 0,1 und 5 % (w/w) beträgt.

6.  Endoprothesekörper gemäß einem der Ansprüche 1 bis 5, wobei der Gehalt an einem oder mehreren superreinen RE in der Legierung zwischen 0,1 und 5 % (w/w) beträgt.

7.  Endoprothesekörper gemäß einem der Ansprüche 1 bis 6, wobei das Konstruktionsmaterial eine Korngröße von weniger als 5 Mikrometer aufweist.

8.  Endoprothesekörper gemäß einem der Ansprüche 1 bis 7, der ein medizinischer Stent ist.

9.  Endoprothesekörper gemäß einem der Ansprüche 1 bis 7, der ein Arzneimittel-eluierender medizinischer Stent ist.

10. Endoprothesekörper gemäß einem der Ansprüche 1 bis 7, der eine medizinische Klammer ist.

11. Endoprothesekörper gemäß einem der Ansprüche 1 bis 7, der ein medizinischer Bolzen ist.

12. Endoprothesekörper gemäß einem der Ansprüche 1 bis 7, der eine medizinische Platte ist.

13. Endoprothesekörper gemäß einem der Ansprüche 1 bis 7, der eine medizinische Wendel ist.

14. Endoprothesekörper gemäß einem der Ansprüche 1 bis 7, der ein Röntgenmarker ist.

15. Endoprothesekörper gemäß einem der Ansprüche 1 bis 7, der ein medizinischer Katheter ist.

16. Endoprothesekörper gemäß einem der Ansprüche 1 bis 7, der eine medizinische Schraube, ein medizinisches Schlauchnetz, ein medizinischer Draht oder eine medizinische Spirale ist.

17. Verwendung eines Konstruktionsmaterials gemäß einem der Ansprüche 1 bis 7 für die Herstellung eines Endoprothesekörpers gemäß einem der Ansprüche 8 bis 16.

18. Konstruktionsmaterial umfassend superreines Magnesium gemäß Anspruch 1 und eine oder mehrere superreine Legierungselemente gemäß einem der Ansprüche 1 bis 7.

19. Verfahren zum Herstellen eines Konstruktionsmaterials für einen medizinischen bioabbaubaren Endoprothesekörper, umfassend den Schritt des Kombinierens von superreinem Magnesium gemäß Anspruch 1 und einem oder mehreren superreinen Legierungselementen gemäß Anspruch 1, um eine Legierung zu bilden.

20. Verfahren gemäß Anspruch 19, wobei das eine oder die mehreren superreinen Legierungselemente in der Legierung in der Menge gemäß einem der Ansprüche 2 bis 6 kombiniert werden.

**Revendications**

1. Corps d'endoprothèse médicale biodégradable, formé au moins partiellement à partir d'un matériau de construction dans un alliage comprenant du magnésium ultrapur et un ou plusieurs éléments d'alliage ultrapurs, dans lequel

   - le magnésium ultrapur a une pureté supérieure ou égale à 99,998 % (pds/pds) et contient un niveau d'impuretés de fer, cobalt, nickel et cuivre, chacun égal ou inférieur à 0,0002 % (pds/pds) de chaque dite impureté ;
   - le ou les éléments d'alliage ultrapurs ont chacun une pureté supérieure ou égale à 99,99 % (pds/pds) et chacun contient des impuretés de fer, cobalt, nickel et cuivre à un niveau ne dépassant pas 0,00025 % (pds/pds) de chaque dite impureté, le ou les éléments d'alliage ultrapurs étant choisis parmi le scandium, l'yttrium, l'indium, le gallium ou une ou plusieurs terres rares (TR).

2. Corps d'endoprothèse selon la revendication 1, la teneur en scandium ultrapur dans l'alliage se situant entre 0,1 et 15 % (pds/pds).

3. Corps d'endoprothèse selon la revendication 1 ou 2, la teneur en yttrium ultrapur dans l'alliage se situant entre 0,1 et 5 % (pds/pds).

4. Corps d'endoprothèse selon l'une quelconque des revendications 1 à 3, la teneur en indium ultrapur dans l'alliage se situant entre 0,1 et 5 % (pds/pds).

5. Corps d'endoprothèse selon l'une quelconque des revendications 1 à 4, la teneur en gallium ultrapur dans l'alliage se situant entre 0,1 et 5 % (pds/pds).

6. Corps d'endoprothèse selon l'une quelconque des revendications 1 à 5, la teneur de la ou des TR ultrapures dans l'alliage se situant entre 0,1 et 5 % (pds/pds).

7. Corps d'endoprothèse selon l'une quelconque des revendications 1 à 6, le matériau de construction ayant une grosseur de grain inférieure à 5 $\mu$m.

8. Corps d'endoprothèse selon l'une quelconque des revendications 1 à 7, qui est un stent médical.

9. Corps d'endoprothèse selon l'une quelconque des revendications 1 à 7, qui est un stent médical à élution de médicaments.

10. Corps d'endoprothèse selon l'une quelconque des revendications 1 à 7, qui est une agrafe médicale.

11. Corps d'endoprothèse selon l'une quelconque des revendications 1 à 7, qui est un boulon médical.

12. Corps d'endoprothèse selon l'une quelconque des revendications 1 à 7, qui est une plaque médicale.

13. Corps d'endoprothèse selon l'une quelconque des revendications 1 à 7, qui est une bobine médicale.

14. Corps d'endoprothèse selon l'une quelconque des revendications 1 à 7, qui est un marqueur aux rayons X.

15. Corps d'endoprothèse selon l'une quelconque des revendications 1 à 7, qui est un cathéter médical.

16. Corps d'endoprothèse selon l'une quelconque des revendications 1 à 7, qui est une vis médicale, un treillis tubulaire médical, un fil médical ou une spirale médicale.

17. Utilisation d'un matériau de construction tel que défini dans l'une quelconque des revendications 1 à 7 pour la fabrication d'un corps d'endoprothèse tel que défini dans l'une quelconque des revendications 8 à 16.

18. Matériau de construction comprenant du magnésium ultrapur tel que défini dans la revendication 1 et d'un ou plusieurs éléments d'alliage ultrapurs tels que définis dans l'une quelconque des revendications 1 à 7.

19. Procédé de fabrication d'un matériau de construction pour un corps d'endoprothèse médicale biodégradable, comprenant l'étape de combinaison de magnésium ultrapur tel que défini dans la revendication 1 et d'un ou plusieurs éléments d'alliage ultrapurs tels que définis dans la revendication 1 pour former un alliage.

20. Procédé selon la revendication 19, dans lequel le ou les éléments d'alliage ultrapurs sont combinés dans l'alliage dans la quantité définie dans l'une quelconque des revendications 2 à 6.

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- US 5698158 A
- US 6287332 B
- US 20020004060 A1
- US 20090076596 A
- US 20090198320 A1
- EP 1835042 A1
- US 2004098108 A, H. Claus; G. Bodo; H. Mueller; B. Heublein
- EP 2000551 A
- US 20040241036 A1
- EP 1835043 A1

## Non-patent literature cited in the description

- **PEUSTER M ; FINK C ; WOHLSEIN P ; BRUEGMANN M ; GUNTHER A ; KAESE V et al.** Degradation of tungsten coils implanted into the subclavian artery of New Zealand white rabbits is not associated with local or systemic toxicity. *Biomaterials,* 2003, vol. 24, 393-399
- **M. PEUSTER ; P. WOHLSEIN ; M. BRÜGMANN ; M. EHLERDING ; K. SEIDLER ; C. FINK ; H. BRAUER ; A. FISCHER ; G. HAUSDORF.** A novel approach to temporary stenting: degradable cardiovascular stents produced from corrodible metal-results 6-18 months after implantation into New Zealand white rabbits. *Heart,* 2001, vol. 86, 563-569
- **WAKSMAN R ; PAKALA R ; BAFFOUR R ; SEABRON R ; HELLINGA D ; FERMIN O.** Short-term effects of biocorrodible iron stents in porcine coronary arteries. *J Interv Cardiol,* 2008, vol. 21, 15-20
- **H. HERMAWAN ; D. DUBE ; D. MANTOVANI.** Development of degradable Fe-35Mn Alloy for biomedical application. *Adv Mater Res,* 2007, vol. 15-17, 107-112
- **VORMANN J.** Magnesium: nutrition and metabolism. *Mol Aspects Med,* 2003, vol. 24, 27-37
- **H. ZREIQAT ; C.R. HOWLETT ; A. ZANNETTION ; P. EVANS ; G. SCHULZE-TANZIL ; C. KNABE.** *J. Biomed. Mater. Res.,* 2002, vol. 62, 175
- Magnesium and magnesium alloys - wrought magnesium alloys. *International Standard Organization,* 2007
- Magnesium and magnesium alloys. *Magnesium alloy ingots and castings,* 1997
- Standard practice for codification of certain nonferrous metals and alloys, cast and wrought. Annual book of ASTM standards. American Society for Testing and Materials, 2005
- **B. HEUBLEIN ; R. ROHDE ; V. KAESE ; M. NIEMEYER ; W. HARTUNG ; A. HAVERICH.** Biocorrosion of magnesium alloys: a new principle in cardiovascular implant technology?. *Heart,* 2003, vol. 89, 651-656
- **PIETAK A ; MAHONEY P ; DIAS GJ ; STAIGER MP.** Bone-like matrix formation on magnesium and magnesium alloys. *J Mater Sci Mater Med,* 2008, vol. 19 (1), 407-415
- **MULLER WD ; NASCIMENTO ML ; ZEDDIES M ; CORSICO M ; GASSA LM ; LORENZO DE MELE MAF.** Magnesium and its alloys as degradable biomaterials corrosion studies using potentiodynamic and EIS electrochemical techniques. *Mater Res,* 2007, vol. 10, 5-10
- **WITTE F ; KAESE V ; SWITZER H ; MEYER-LINDENBERG A ; WIRTH CJ ; WINDHAG H.** In vivo corrosion of four magnesium alloys and the associated bone response. *Biomaterials,* 2005, vol. 26, 3557-3563
- **LIU CL ; XIN YC ; TANG GY ; CHU PK.** Influence of heat treatment on degradation behavior of biodegradable die-cast AZ63 magnesium alloy in simulated body fluid. *Mater Sci Eng A,* 2007, vol. 456, 350-357
- **KANNAN MB ; RAMAN RKS.** In vitro degradation and mechanical integrity of calcium-containing magnesium alloys in modified-simulated body fluid. *Biomaterials,* 2008, vol. 29, 2306-2314
- **WITTE F ; FISCHER J ; NELLESEN J ; CROSTACK H ; KAESE V ; PISCHD A et al.** In vitro and in vivo corrosion measurements of magnesium alloys. *Biomaterials,* 2006, vol. 27, 1013-1018
- **MARIO C ; GRIFFITHS H ; GOKTEKIN O ; PEETERS N ; VERBIST J ; BOSIERS M et al.** Drugeluting bioabsorbable magnesium alloys. *J Interv Cardiol,* 2004, vol. 17 (6), 391-5

- **ZARTNER P ; CESNJEVAR R ; SINGER H ; WEYAND M.** First successful implantation of a biodegradable metal stent into the left pulmonary artery of a preterm baby. *Catheter Cardiovasc Interv,* 2005, vol. 66, 590-4
- **WAKSMAN R ; PAKALA R ; KUCHULAKANTI PK ; BAFFOUR R ; HELLINGA D ; SEABRON R et al.** Safety and efficacy of bioabsorbable magnesium alloy stents in porcine coronary arteries. *Catheter Cardiovasc Interv,* 2006, vol. 68, 607-17
- **ERBEL R ; DI MARIO C ; BARTUNEK J et al.** Temporary scaffolding of coronary arteries with bioabsorbable magnesium stents: a prospective, non-randomised multicentre trial. *Lancet,* 2007, vol. 369 (9576), 1869-1875
- **R. WAKSMAN.** Biodegradable stents: They do their job and disappear. *Journal of Invasive Cardiology,* 2006, vol. 18 (2), 70-74
- **R. WAKSMAN.** Promise and Challenges of Bioabsorbable Stents. *Catheterization and Cardiovascular Interventions,* 2007, vol. 70, 407-414
- **CHRIS KAISER.** Biodegradable stents could be the ideal stent. Healthcare Tech Guide. *Coronary Stents Portal,* 08 March 2009
- **XU L ; YU G ; ZHANG E ; PAN F ; YANG K.** In vivo corrosion behavior of Mg-Mn-Zn alloy for bone implant application. *J Biomed Mater Res,* 2007, vol. 83A (3), 703-11
- **Z, GU X ; LOU S ; ZHENG Y.** The development of binary Mg-Ca alloys for use as biodegradable materials within bone. *Biomaterials,* 2008, vol. 29, 1329-44
- Investigation of the mechanical properties and the corrosion behaviour of low alloyed magnesium-calcium alloys for use as absorbable biomaterial in the implant technique. **T. HASSEL ; FR.-W. BACH ; A. GOLOVKO ; C. KRAUSE.** Magnesium Technology in the Global Age, 45th Annual Conference of Metallurgists of CIM, Montreal, Quebec, Canada. 2006, 359-370
- **O.V.CHERNYI ; N.F.ANDRIEVSKAYA ; V.O.ILICHEVA ; G.E.STOROZHILOV ; P.J.LEE ; A.A.SQUITIERI.** The Microstructure and Critical Current Density of Nb-48wt.%Ti Superconductor with Very High Alpha-Ti Precipitate Volume and Very High Critical Current Advances. *Cryogenic Engineering,* 2002, vol. 48B, 883-890
- **V.E. IVANOV ; I.I. PAPIROV et al.** Pure and high-pure metals. *M. Metallurgija,* 1965, 152
- **A.I. BORISKIN ; Y.S.BRUCHANOV.** Mass-spectrometers with double focusing and a laser source of ions. *Science Devices,* 1981, 28-30
- **A.M. LIPNITSKY ; I.V. MOROZOV.** Technology of nonferrous castings. *L: Mashgiz,* 1986, 224